# EUROPÄISCHE PATENTSCHRIFT

(11) **EP 0 564 620 B1**
(45) Veröffentlichungstag und Bekanntmachung des Hinweises auf die Patenterteilung: **03.03.1999**
(21) Anmeldenummer: 92921537.4
(22) Anmeldetag: 17.10.1992
(51) Int. Cl.: A61K 39/00, C12N 1/14

(54) **DERMATOMYKOSE-VAKZINE**
DERMATOMYCOSIS VACCINE
VACCINS CONTRE LES DERMATOMYCOSES

(30) Priorität: 21.10.1991 SU 5006861
(43) Veröffentlichungstag der Anmeldung: 13.10.1993
(73) Patentinhaber: BOEHRINGER INGELHEIM VETMEDICA GMBH, 55216 Ingelheim (DE)
(72) Erfinder: POLYAKOV, Igor, Dimitriesich, Moskau, 115682 (RU); IVANOVA, Ludmilla, Moskau, 115682 (RU)
(86) Internationale Anmeldenummer: EP9202391
(87) Internationale Veröffentlichungsnummer: WO9307894

(56) Entgegenhaltungen:
- EP-A- 0 393 371
- SU-A- 1 177 972
- BIOLOGICAL ABSTRACTS vol. 81, no. 1 , 1986, Philadelphia, PA, US; abstract no. 4165, A. SARKISOV 'SPECIFIC PROPHYLAXIS OF TRICHOPHYTOSIS IN ANIMALS.' Seite AB-467 ;
- BIOLOGICAL ABSTRACTS vol. 92, no. 11 , 1. Dezember 1991, Philadelphia, PA, US; abstract no. 124650, J. WAWRZKIEWICZ ET AL. 'MONOVALENT AND COMBINED INACTIVATED (KILLED) VACCINES IN THE PROPHYLAXIS OF TRICHOPHYTOSIS OF BREEDING FOXES.' Seite AB-556 ;

## Beschreibung

Die vorliegende Erfindung betrifft die Bereitstellung von Impfstoffen und ihre Verwendung zur Herstellung von Mitteln zur spezifischen Prävention und Behandlung von Dermatomykosen.

Dermatomykosen an Tieren sind anthropozoonotische Krankheiten der Haut und der damit verbundenen Gewebe. Klinische Symptome sind durch Haarverlust in den betroffenen Bereichen, Hyperämie, schuppenartigen und Asbest-artigen Schorf gekennzeichnet. Entzündungen gehen oft einher mit Suppuration. Außerdem sind Dermatomykosen oft durch lokale Infektionen der Haut gekennzeichnet.

Dermatomykosen an Tieren besitzen eine beträchtliche sozioökonomische Bedeutung. Erkrankte Tiere benötigen eine langandauernde Behandlung und können die Infektion sowohl auf Tiere als auch auf Menschen übertragen.

Bis jetzt werden Dermatomykosen durch die Verwendung verschiedenster Medikamententypen behandelt, die lokal auf die betroffenen Bereiche der Haut aufgetragen werden. Sie schließen die Salben YaM, Yuglon (1) und eine Vielzahl anderer Salben, Einreibemittel, Lösungen und andere Substanzen ein, die fungizide und fungistatisch wirkende Mittel enthalten.

Die Nachteile solcher Behandlungen sind:
- ihre geringe Effiktivität;
- sie setzen die Anwendung von Quarantäne-Maßnahmen und die Desinfektion der Bereiche voraus, in denen die Tiere leben (Aufzuchtställe, Tiergehege, Farmen, Zoos, Zirkusse, usw.);
- sie sind kostenintensiv im Hinblick auf die Medikamente und die Tierarztbehandlung
- sie werfen Probleme bei der Ruhigstellung der Tiere auf (wilde Tiere in Käfigen).

Später wurden Impfstoffe zur Behandlung der Trichophytie an Rindern (UdSSR Patent Nr. 268593, 1970), Pelztieren und Kaninchen (UdSSR Patent Nr. 835446, 1980), Kamelen (UdSSR Patent Nr. 1190574, 1985) und andere entwickelt.

Ein Impfstoff zur Prävention und für die Behandlung von Trichophytie in Pferden wurde ebenfalls bereits früher entwickelt: S-P-I (UdSSR Patent Nr. 548947, 1976)(2).

Der S-P-I-Impfstoff enthält den Vakzine-Stamm Trichophyton equinum Nr. 2251/71, hinterlegt beim "USSR All-Union State Scientific Control Institute of Veterinary Preparations", der in Agar/Bierwürze für 20 bis 25 Tage bei einer Temperatur von 26 bis 28°C angezogen wird. Die Pilzmasse wird dann von der Oberfläche abgehoben, mit sterilem, destilliertem Wasser gemischt, homogenisiert und die Konzentration an Zellen auf 600 bis 900 Millionen pro Milliliter eingestellt. Das Homogenat wird dann in eine separate Flasche überführt und mit einer Mischung, die 2 bis 8 % Gelantine (Gelatose) und 10 bis 40 % Sucrose enthält, im Verhältnis 1:1 (+/- 25 %) stabilisiert und dann lyophilisiert.

Für prophylaktische und therapeutische Zwecke wird der Impfstoff in das Muskelgewebe des Nackenbereiches von jungen und ausgewachsenen Pferden in Zwei Dosen von 1 bis 2 cm³ in Abhängigkeit vom Alter des Pferdes in einem Intervall von 10 bis 14 Tagen injiziert. Zur Therapie werden doppelte Dosen verwendet.

Impfstoffe, die nach dieser Methode erhalten werden, besitzen den Nachteil, daß sie keine Immunität gegen Mikrosporie und Trichophytie vermitteln, die durch andere Agentien verursacht werden. Es muß auch berücksichtigt werden, daß Gebiete, in denen Lebendvakzine verwendet werden, zu spezifischen Krankheitsherden werden können, in denen Kulturen der Impfstoff-Stämme zu bestimmten Zeiten gebildet werden. Impfstoff-Stämme besitzen eine Restvirulenz. Falls Haustierspezies in häufigem Kontakt mit Menschen kommen, ist das Auftreten solcher spezifischer Herde nicht akzeptierbar.

"Die EP 393371 zitiert allgemein 22 Dermatophyten-Spezies aus dem "Medical Mycology Handbook"_{,} Campbell and Stewart, 1980, als Erreger von Dermatomykosen. Epizootische Kulturen dieser Dermatophyten-Spezies sollen in Flüssigmedium kultiviert, mit Formalin inaktiviert, anschließend homogenisiert und mit einem Adjuvant versetzt als Vakzine verwendet werden können.

Als Arbeilsbeispiel für eine monovalente Vakzine wird eine inaktivierte M. canis Kultur angegeben. Als Arbeitsbeispiel für polyvalente Vakzine werden zwei, ebenfalls M. canis enthaltende, trivalente Vakzine, bestehend aus den mit Formalin inaktivierten, homogenisierten epizootischen Dermatophyten M. canis, M. gypseum und T. mentagrophytes, oder M. canis, M. gypseum und einer nicht näher definierten Spezies der Gattung Alternaria, beschrieben.

Weitere polyvalente Vakzine auf der Basis vorteilhafter Kombinationen epizootischer Stämme, werden in der EP 393 371 nicht beschrieben."

Die vorliegende Erfindung stellt nun universale Tot-Impfstoffe zur spezifischen Behandlung und Prävention von Dermatomykosen an Tieren und entsprechende immunogene Pilzstämme zur Verfügung.

Die Erfindung wurde durch die Verwendung folgender Pilzstämme als Impfstoffstämme verwirklicht. Trichophyton verrucosum, insbesondere Trichophyton verrucosum Nr. VKPGF-931/410), T. mentagrophytes, insbesondere T. mentagrophytes Nr. VKPGF-930/1032, T. equinum, insbesondere T. equinum Nr. VKPGF-929/381,
Microsporum canis, insbesondere M. canis Nr. VKPGF-928/1393, M. canis var. obesum, insbesondere M. canis var. obesum Nr. VKPGF-727/1311, M. canis var. distortum, insbesondere M. canis var. distortum Nr. VKPGF-728/120, M. gypseum, insbesondere M. gypseum Nr. VKPGF-729/59. Impfstoffe können durch verschiedene Kombinationen von antigenem Material der oben genannten Stämme und einem geeigneten Träger hergestellt werden.

Eine bevorzugte Kombination ist dabei Trichopyhton verrucosum Nr. VKPGF-931/410, Trichophyton mentagrophytes Nr. VKPGF-930/1032, Trichophyton equinum Nr. VKPGF-551/68, Microsporum canis Nr. VKPGF-928/1393, Microsporum canis var. obesum Nr. VKPGF-727/1311, Microsporum canis var. distortum Nr. VKPGF-728/120 und Microsporum gypseum Nr. VKPGF-729/59 in Frage, insbesondere für Hund, Katze und Pferd.

Eine bevorzugte Kombination an Impfstoffstämmen ist auch Trichophyton verrucosum Nr. VKPGF-931/410, Trichophyton mentagrophytes Nr. VKPGF-920/1032 und Trichophyton sarkisovii Nr. VKPGF-551/68, insbesondere für die Anwendung bei Rindern.

Das antigene Material kann ein einzelnes Antigen von mindestens einem, insbesondere von allen der oben genannten Dermatophyten oder einer Vielzahl von Antigenen umfassen, solange eine ausreichende Immunantwort stimuliert wird, die eine Resistenz gegen eine Dermatophyteninfektion bewirkt. Antigenes Material für eine solche Verwendung kann mit aus dem Stand der Technik bekannten Verfahren hergestellt werden, z.B. Homogenisation der genannten Dermatophyten oder Teilen der Dermatophyten, Fraktionierung von Dermatophytenpräparationen, Produktion von antigenen Dermatophytenmaterial durch rekombinante DNA Technologie usw.. Bevorzugt kann homogenisiertes Kulturmaterial mit 40 bis 120 Millionen, bevorzugt 90 Millionen Mikrokonidien verwendet werden.

Geeignete physiologisch akzeptable Träger für die Verabreichung der Impfstoffe sind aus dem Stand der Technik bekannt und können Puffer, Gele, Mikropartikel, implantierbare Feststoffe, Lösungen und andere Adjuvantien umfassen.

Zur Abtötung der Dermatophyten kann Thiomersal C₉H₉O₂SNaHg), Formaldehyd oder 2-Propyolacton verwendet werden.

Zur Herstellung eines Impfstoffes kann zum Beispiel folgendermaßen vorgegangen werden:

Kulturen der Stämme werden in einer wäßrigen Lösung mit 0,2 bis 2,0 % fermentiertem, hydrolysiertem Muskelprotein (FGM-s), 5 bis 12 % Glukose und 0,1 bis 1,2 % Hefeextrakt homogenisiert. Die Konzentration der Mikrokonidien wird auf 40 bis 120 Millionen pro Milliliter eingestellt und die Mischung nach 1 bis 2 Tagen mit z.B. mit Thiomersal (C₉H₉O₂SNaH_{g}) im Verhältnis 1:10000 bis 1:25000, oder einer anderen aus dem Stand der Technik bekannten Substanz inaktiviert. Die resultierende Suspension wird verpackt und ist fertig zur Verwendung an Tieren.

Die Herstellung der Impfstoffe, die jeweilige Dosis und Form der Verabreichung zur Prävention und therapeutischen Behandlung sind in Beispiel 1 bis 3 erläutert.

Die Erfindung erlaubt nun die Bereitstellung eines inaktivierten Impfstoffes, der die Wahrscheinlichkeit der Reinfektion herabsetzt und außerdem ein hohes Maß an Immunogenität verleiht. Im Gegensatz zu den bekannten Impfstoffen verleiht der erfindungsgemäße Impfstoff in der Praxis Immunität gegen alle wichtigen Ursachen von Dermatomykosen an Tieren.

Kurz zusammengefaßt bietet der erfindungsgemäße Impfstoff folgende Vorteile:
- er etabliert in vielen Spezies von krankheitsanfälligen Tieren Immunität nach intramuskulärer Injektion,
- er etabliert Immunität gegen praktisch alle Ursachen von Dermatomycosen in Tieren,
- er besitzt stabile immunogene Eigenschaften,
- er kann auf einfache Weise hergestellt werden,
- er besitzt einen kompletten Satz an Exo- und Endoantigenen von Dermatophyten Kulturen und zeigt keine Nebenreaktionen in Tieren.

Der Impfstoff wurde erfolgreich an über 500 Tieren verschiedener Spezies, vornehmlich in betroffenen Gebieten, getestet.

Die Stämme, die zur Produktion des Impfstoffes benutzt werden, sind hinterlegt bei der "All-Union Collection of Pathogenic Fungi within the USSR, Ministry of Health Centre for Deep Mycoses" in Leningrad sowie bei der "DSM - Deutsche Sammlung von Mikroorganismen und Zellkulturen", Mascheroder Weg 1B, W-3300 Braunschweig, Deutschland.
* VKPG: Vsesoyuznaya kollektsiya patogennykh gribov (All-Union Collection of Pathogenic Fungi) F : Fungi
Ihre Charakteristika werden im folgenden aufgeführt:

### TRICHOPHYTON VERRUCOSUM, Nr. VKPGF-931/410

Der Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7277 hinterlegt.

Der Stamm wurde mit Hilfe gerichteter Selektion basierend auf Sporenproduktion und Attenuation des epizootischen Stammes Nr. 410, erhalten, der im Jahre 1978 an einem Hirsch gefunden wurde. Der Stamm wurde mit Hilfe des Rebell-Taplin-Schlüssels (Rebell, G., Taplin, D.: Dermatophytes, their recognition and identification, 1978) und nach Kashkin, P.N. et al. (Opredelitel patogennykh, toksigenykh vrednykh dlya cheloveka gribov, 1979) identifiziert.

Die biologischen Eigenschaften des Stammes sind in Tabelle 1 beschrieben.

Stamm-Nr. VKPGF-931/410 unterscheidet sich vom epizootischen Stamm durch sein schnelleres Wachstum in Nährmedium, die enorme Produktion von Mikrokonidien, eine geringere Virulenz und die Abwesenheit einer Reaktion mit seinen Antigenen.

### TRICHOPHYTON MENTAGROPHYTES, Nr, VKPGF-930/1032

Der Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7279 hinterlegt.

Der Stamm wurde mit Hilfe gerichteter Selektion basierend auf Sporenproduktion und Attenuation des epizootischen Stammes Nr. 1032 erhalten, der an einem Pferd im Jahre 1985 gefunden wurde. Der Stamm wurde wie oben angegeben identifiziert (Rebell, Taplin, loc. cit. und Kashkin, loc. cit.). Die biologischen Eigenschaften sind in Tabelle 2 beschrieben.

Der Stamm Nr. VKPGF-930/1032 unterscheidet sich vom epizootischen Stamm durch sein schnelleres Wachstum in Nährmedium, die enorme Produktion an Mikrokonidien, die geringere Virulenz und die Abwesenheit einer Reaktion mit seinen Antigenen.

### TRICHOPHYTON EQUINUM, Nr. VKPGF-929/381

Der Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7276 hinterlegt.

Der Stamm wurde mit Hilfe gerichteter Selektion basierend auf Sporenproduktion und Attenuation des epizootischen Stammes Nr. 381 erhalten, der im Jahre 1986 an einem Pferd gefunden wurde. Er wurde wie oben angegeben identifiziert (Rebell, Tablin, loc. cit. und Kashkin, loc. cit.). Die biologischen Eigenschaften des Stammes sind in Tabelle 3 beschrieben.

Der Stamm Nr. VKPGF-929/381 unterscheidet sich vom epizootischen Stamm durch sein schnelleres Wachstum in Nährmedium, eine geringere Virulenz und die Abwesenheit einer Reaktion mit seinen Antigenen.

### MICROSPORUM CANIS, Nr. VKPGF-928/1393

Der Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7281 hinterlegt.

Der Stamm wurde mit Hilfe gerichteter Selektion basierend auf Sporenproduktion und Attenuation des epizootischen Stammes Nr. 1393 erhalten, der im Jahre 1988 an einer Katze gefunden wurde. Der Stamm wurde wie oben angegeben identifiziert (Rebell, Taplin, loc. cit. und Kashkin, loc. cit.). Die biologischen Eigenschaften des Stammes sind in Tabelle 4 beschrieben.

Der Stamm Nr. VKPGF-928/1393 unterscheidet sich vom epizootischen Stamm durch sein schnelleres Wachstum in Nährmedium, die enorme Kapazität Sporen zu tragen, eine geringere Virulenz und die Abwesenheit einer Reaktion mit seinen Antigenen.

### MIKROSPORUM CANIS VAR, OBESUM, NR. VKPGF-727/1311

Der Stamm wurde bei der DSM - Deutsche Sammlung von Mikroorganismen und Zellkulturen, Mascheroder Weg 1B, W-33oo Braunschweig, Deutschland, am 01.10.1992 unter dem Aktenzeichen DSM 7280 hinterlegt.

Der Stamm wurde mit Hilfe gerichteter Selektion basierend auf Sporenproduktion und Attenuation des epizootischen Stammes Nr. 1311 erhalten, der im Jahre 1986 an einem Tiger gefunden wurde. Der Stamm wurde wie oben angegeben identifiziert (Rebell, Taplin, loc. cit. und Kashkin, loc. cit.). Die biologischen Eigenschaften des Stammes sind in Tabelle 5 beschrieben.

Stamm Nr. VKPGF-727/1311 unterscheidet sich vom epizootischen Stamm durch sein schnelleren Wachstum in Nährmedium, die enorme Kapazität Sporen zu tragen, die geringere Virulenz und die Abwesenheit einer Reaktion mit seinen Antigenen.

### MIKROSPORUM CANIS VAR. DISTORTUM, NR. VKPGF-728/120

Der Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7275 hinterlegt.

Der Stamm wurde mit Hilfe gerichteten Selektion basierend auf Sporenproduktion und Attenuation des epizootischen Stammes Nr. 120 erhalten, die im Jahre 1987 an einem schwarzen Panther gefunden wurde. Der Stamm wurde wie oben angegeben identifiziert (Rebell, Taplin, loc. cit. und Kashkin, loc. cit.). Die biologischen Eigenschaften des Stammes sind in Tabelle 6 angegeben.

Stamm Nr. VKPGF-728/120 unterscheidet sich von dem epizootischen Stamm durch sein schnelleres Wachstum in Nährmedium, die enorme Produktion von Mikrokonidien, die geringere Virulenz und die Abwesenheit einer Reaktion mit seinen Antigenen.

### MICROSPORUM GYPSEUM, Nr. VKPGF-729/59

Der Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7274 hinterlegt.

Der Stamm wurde mit Hilfe gerichteter Selektion basierend auf Sporenproduktion und Attenuation des epizootischen Stammes Nr. 59 erhalten, der im Jahre 1985 an einem Pferd gefunden wurde. Der Stamm wurde wie oben angegeben identifiziert (Rebell, Taplin, loc. cit. und Kashkin, loc. cit.). Die biologischen Eigenschaften des Stammes sind in Tabelle 7 beschrieben.

Der Stamm Nr. VKPGF-729/59 unterscheidet sich von dem epizootischen Stamm durch sein schnelleres Wachstum in Nährmedium, die enorme Produktion von Mikrokonidien, die geringere Virulenz und die Abwesenheit einer Reaktion mit seinen Antigenen.

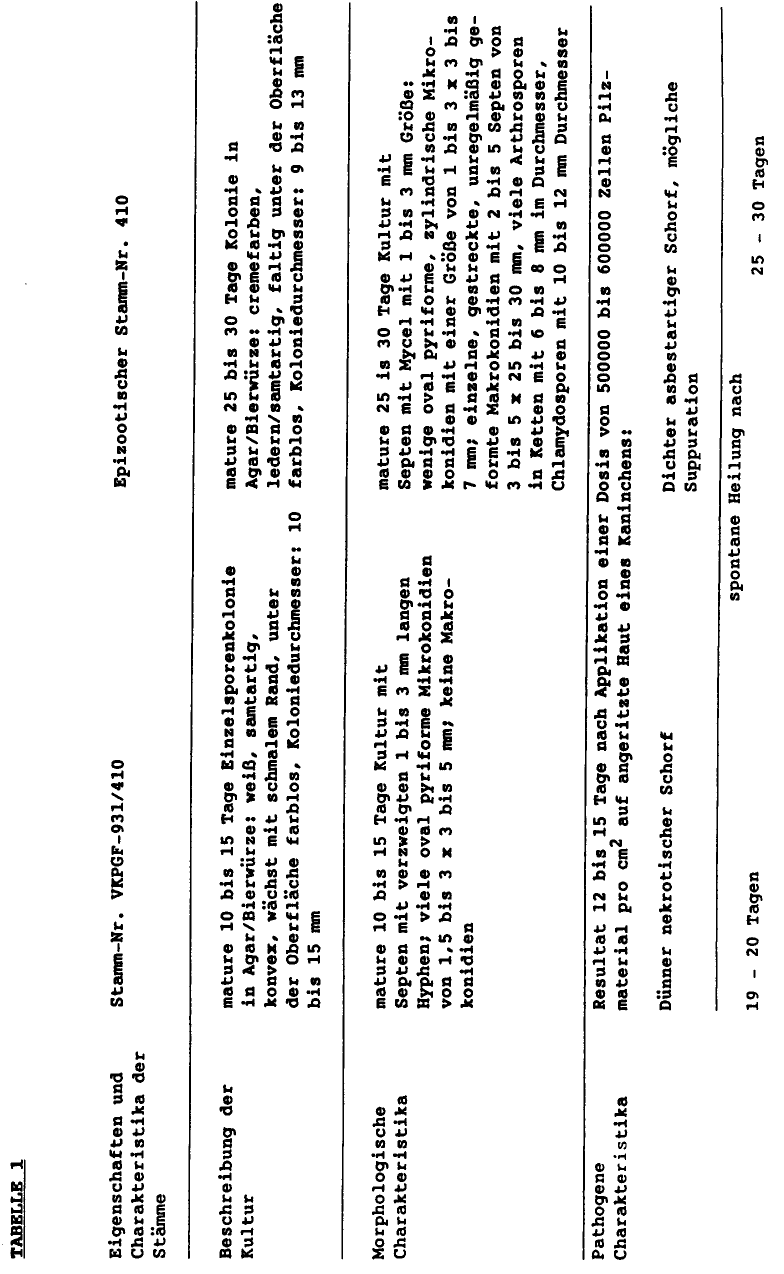

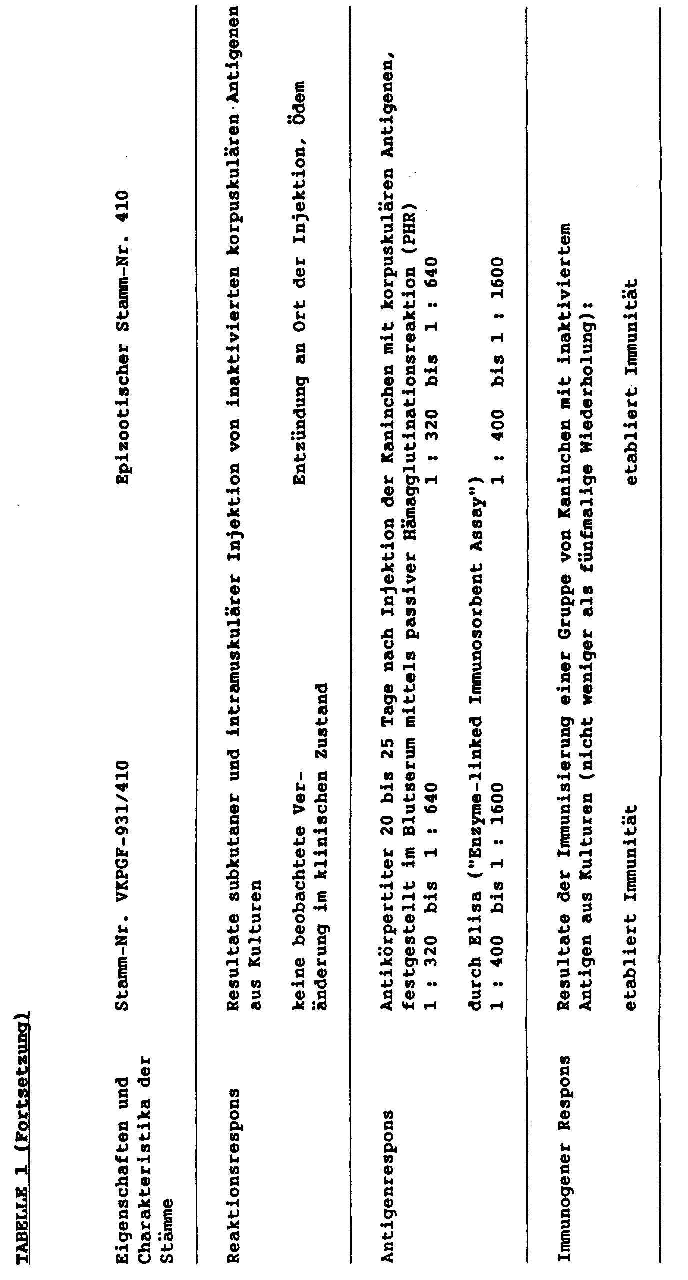

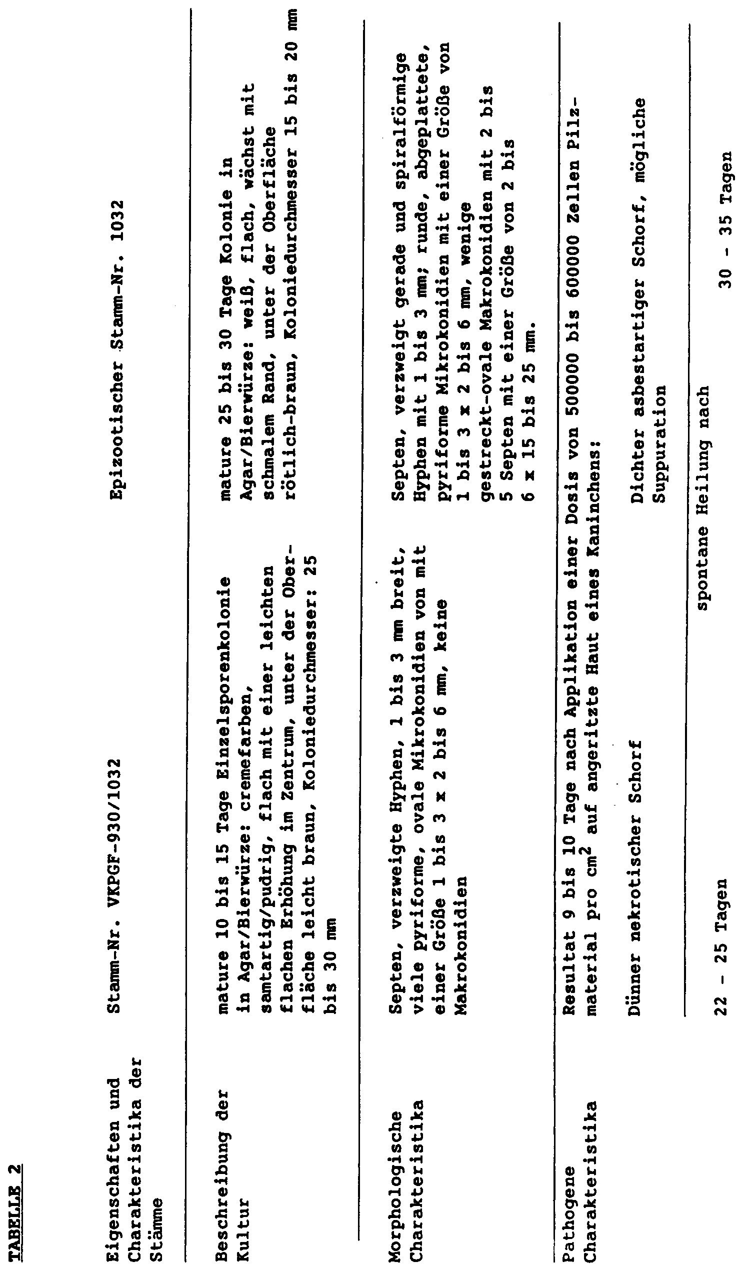

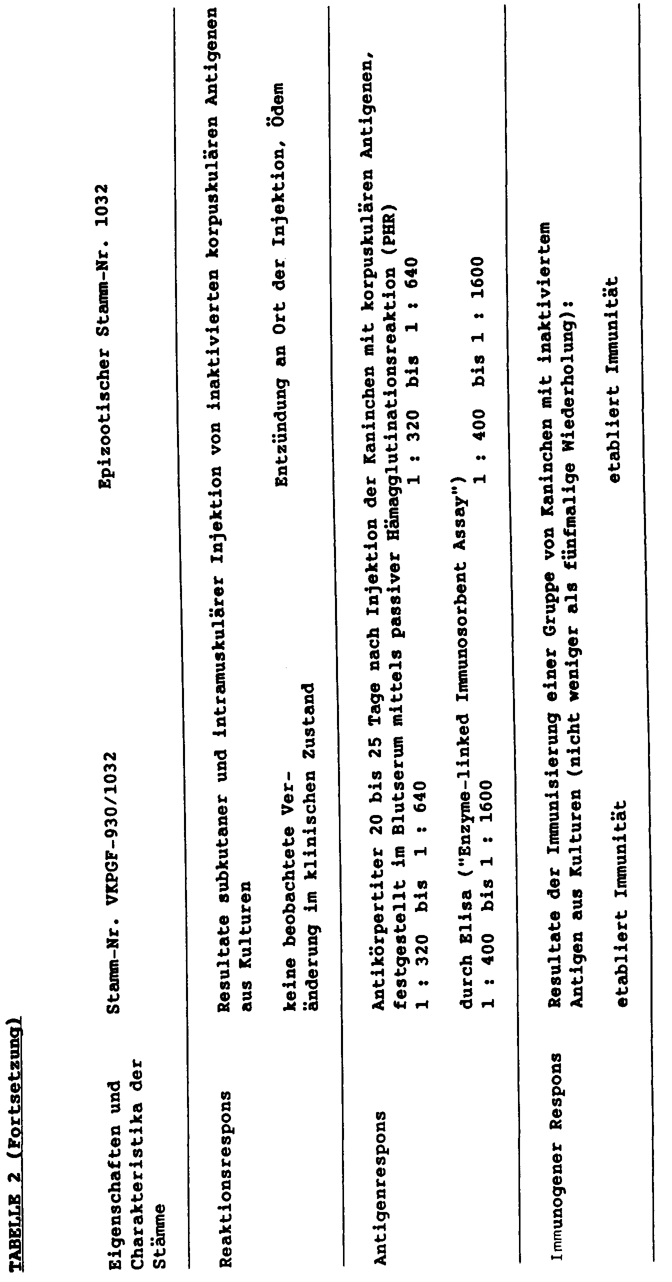

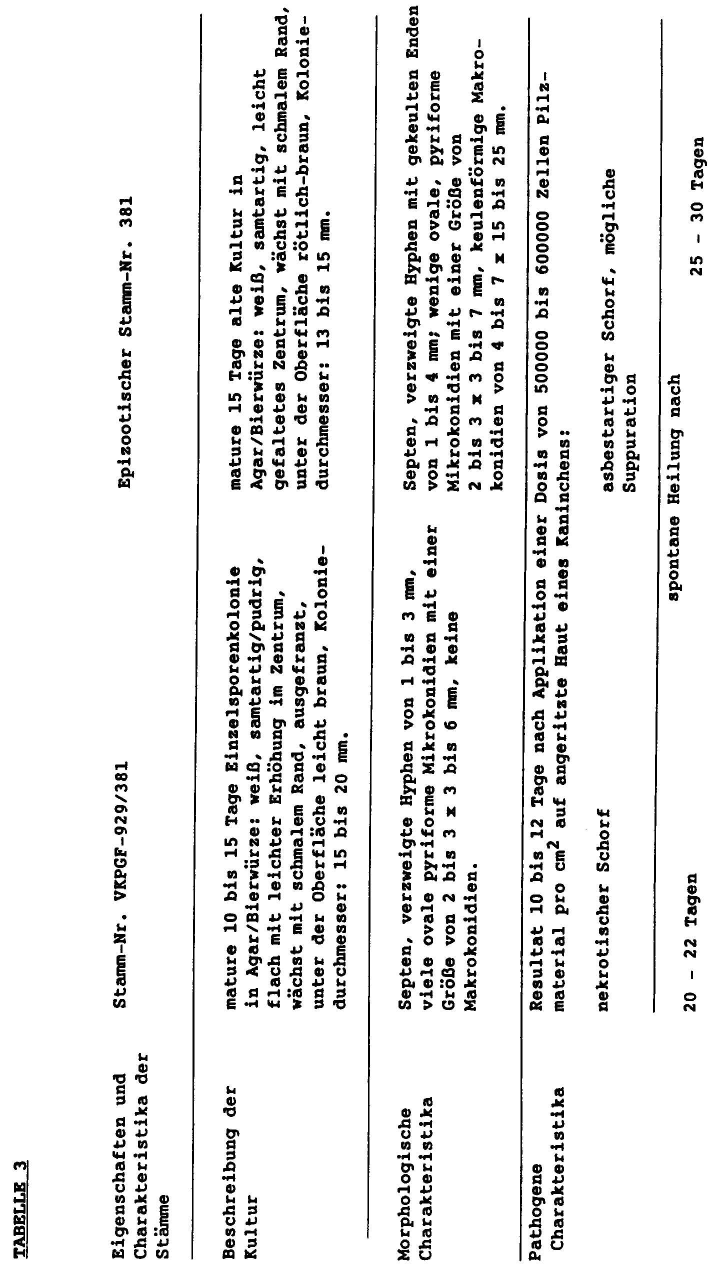

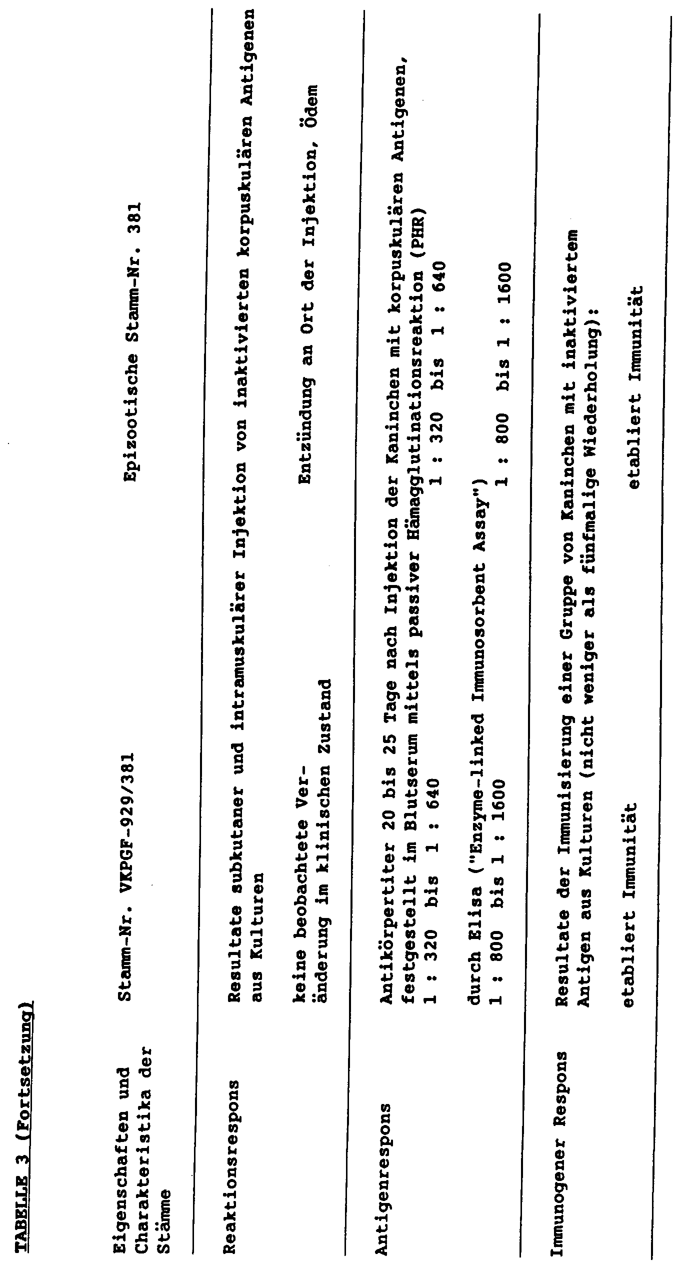

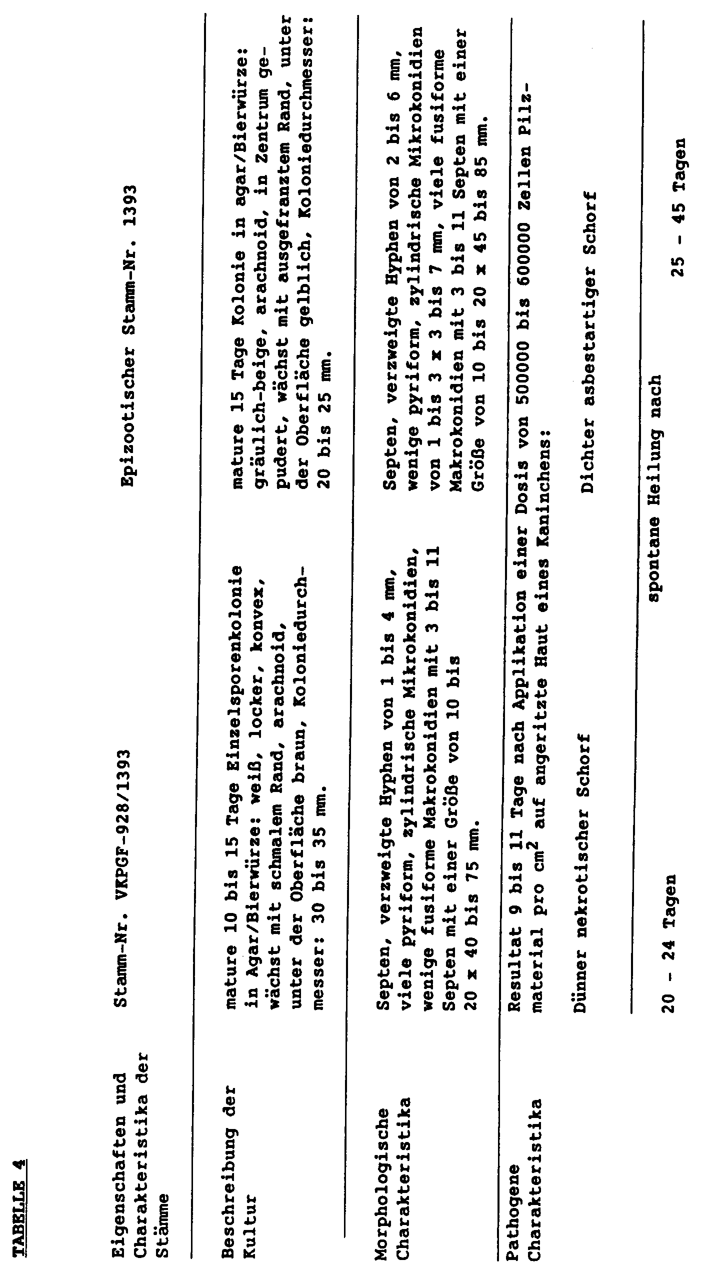

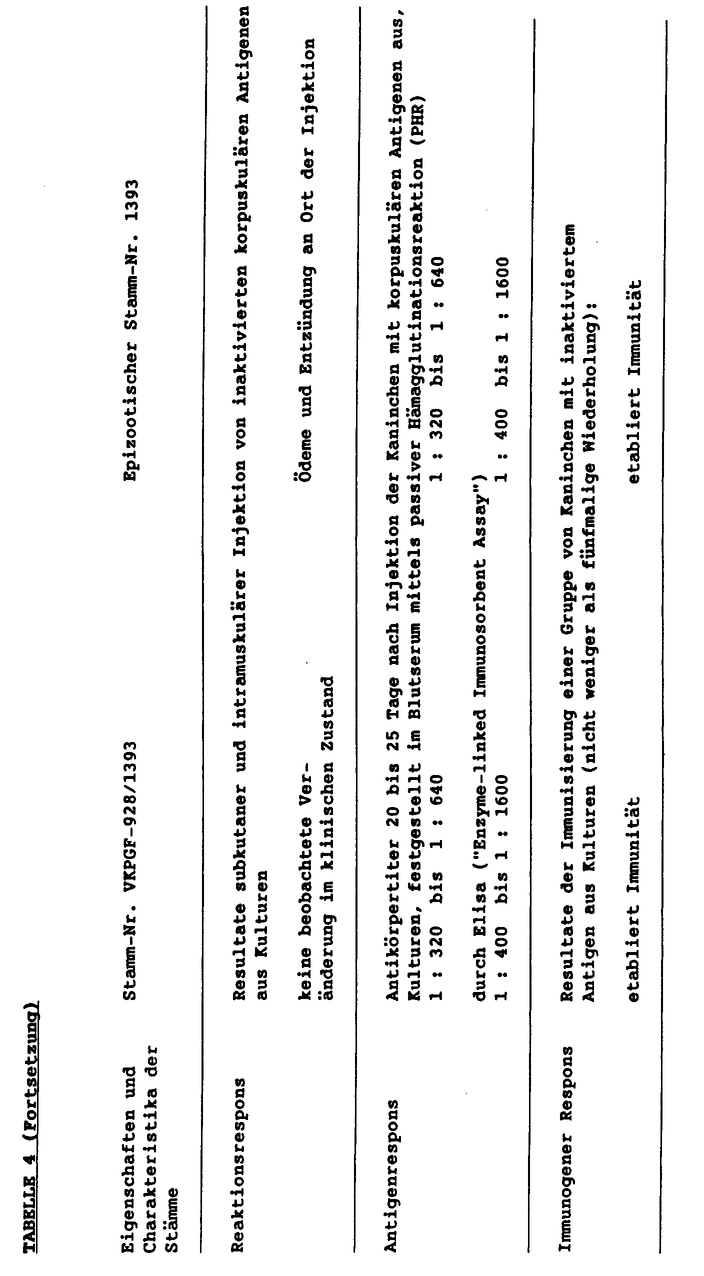

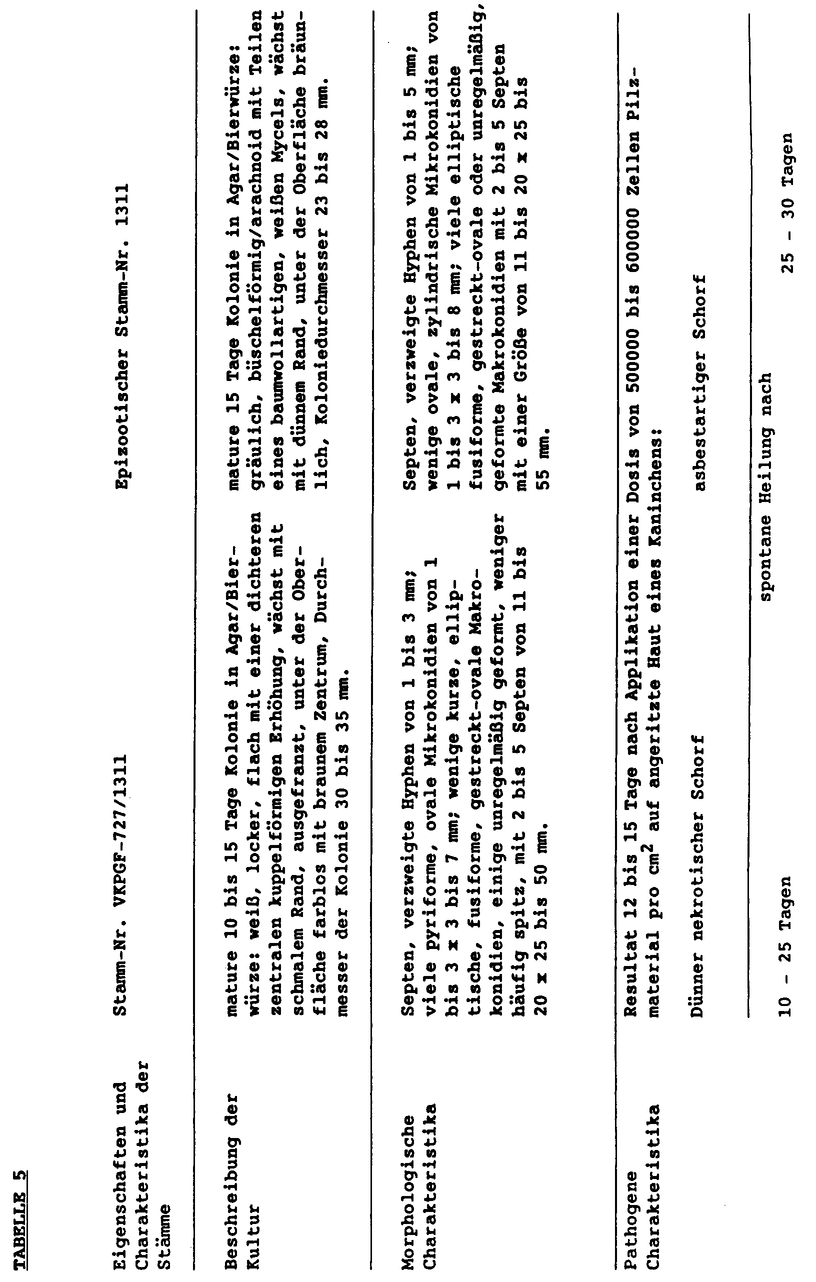

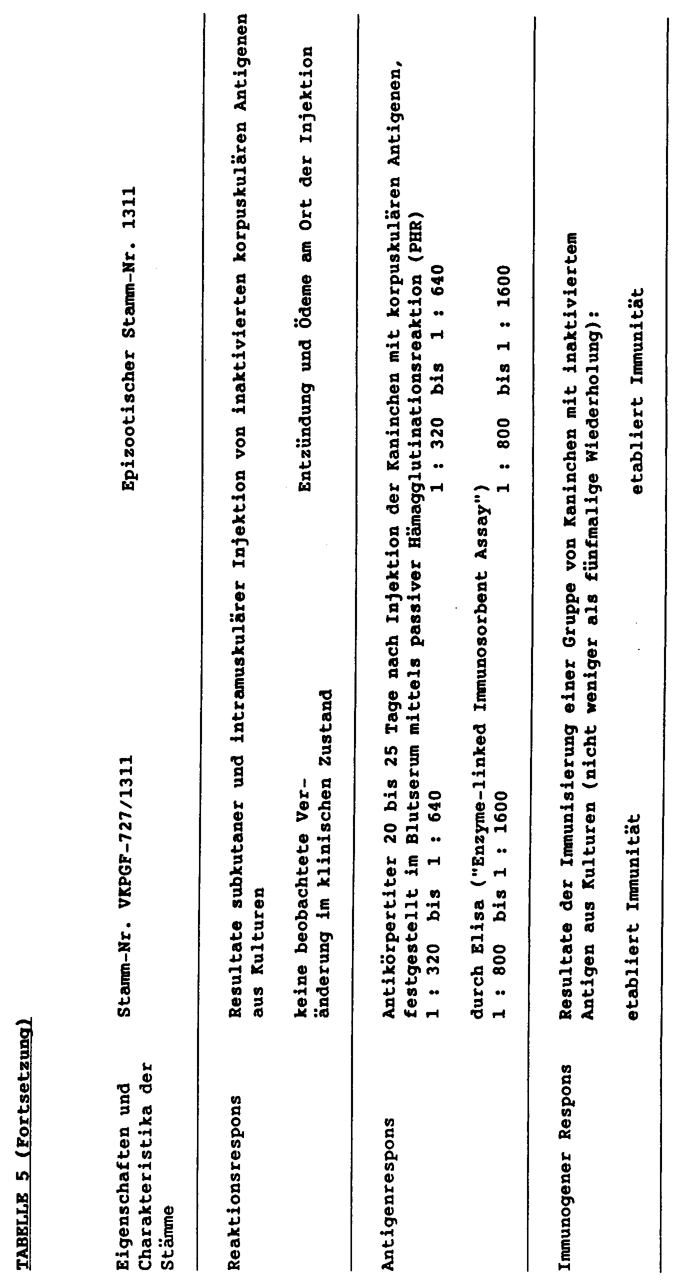

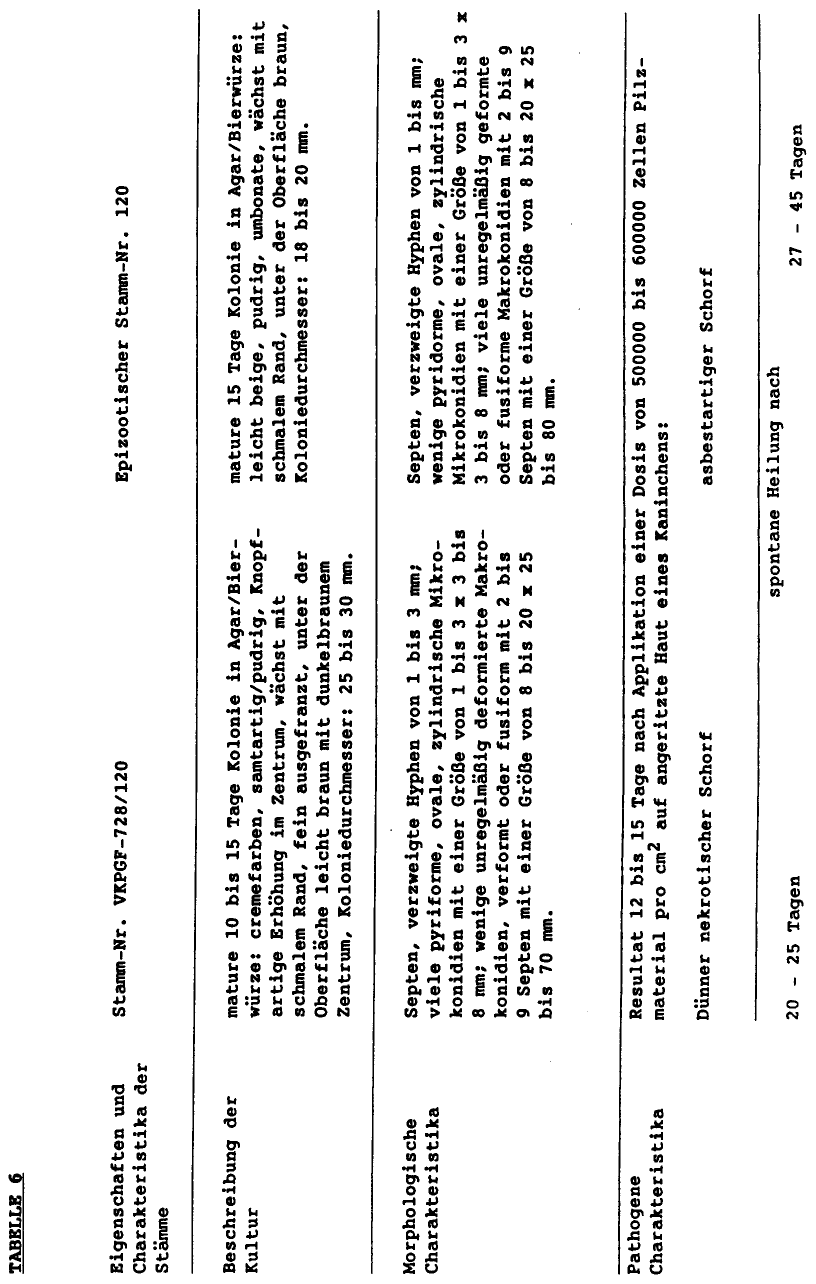

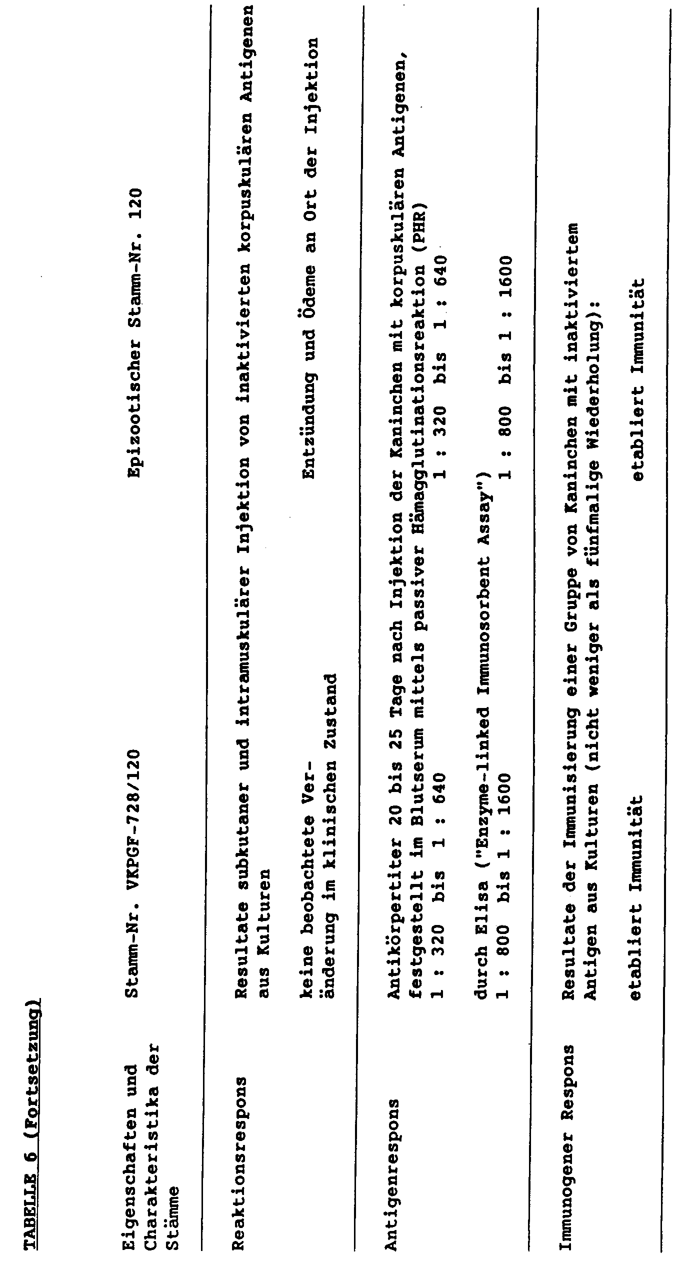

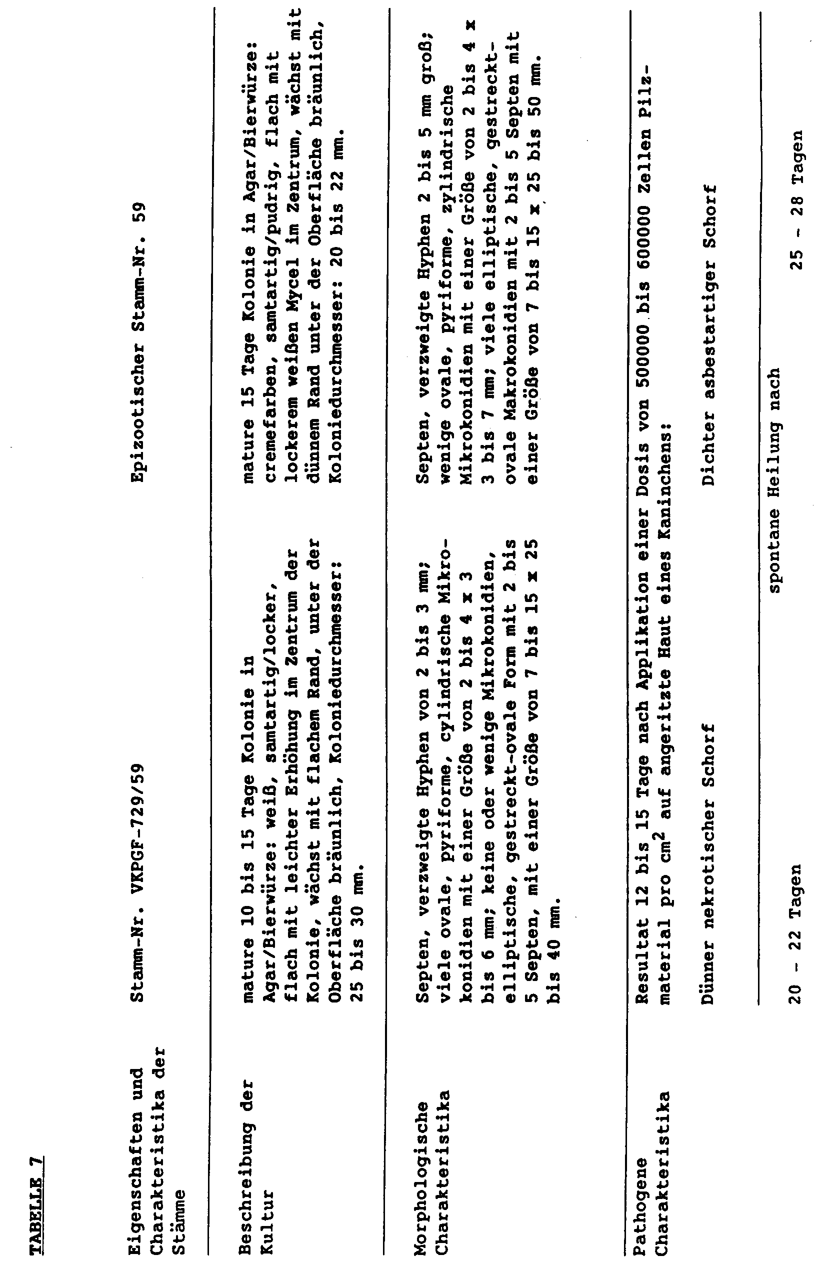

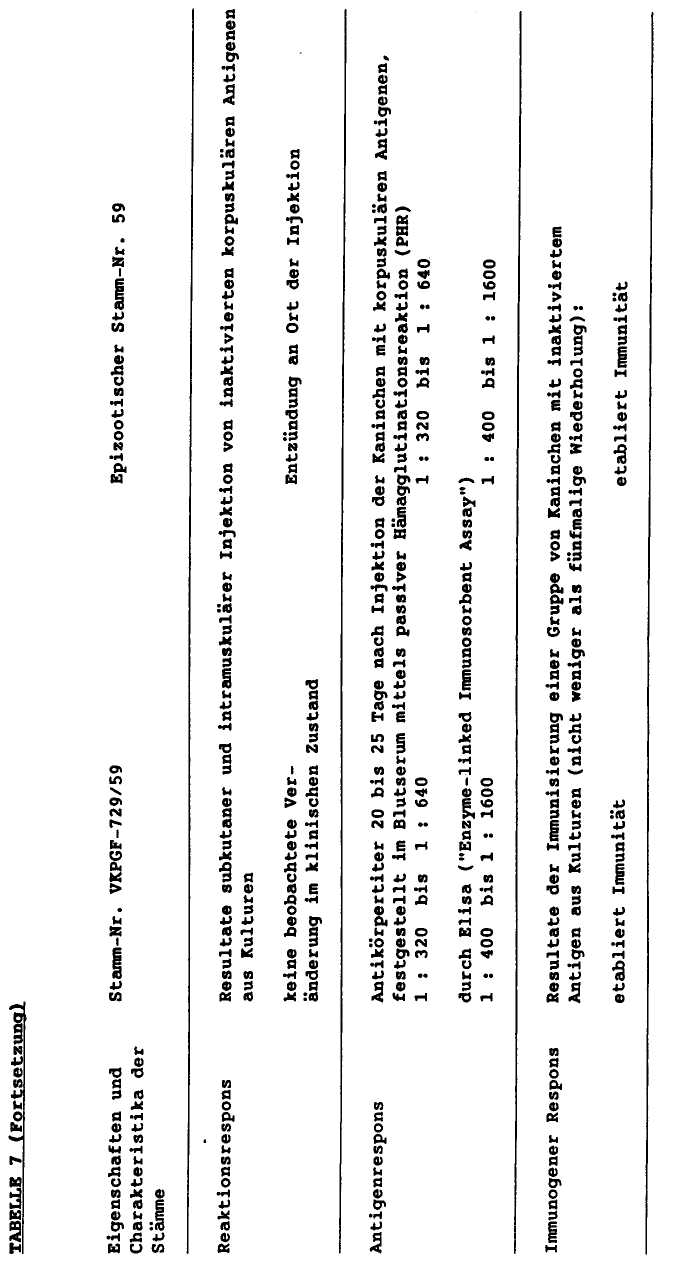

Der Impfstoff kann unter Verwendung des Stammes Trichophyton sarkisovii, Nr. 551/68 hergestellt werden. Er ist z.B. im UdSSR-Patent Nr. 1177972 vom 08.05.1985 beschrieben, Bei dem in diesem Patent beschriebenen Impfstoff handelt es sich um ein Lebendvakzin gegen Trichophytie bei Kamelen. Auch dieser Stamm wurde bei der DSM - Deutsche Sammlung von Mikroorganismen und Zellkulturen, Mascheroder Weg 1B, W-3300 Braunschweig, Deutschland, am 01.10.1992 unter dem Aktenzeichen DSM 7278 hinterlegt.

Im einzelnen umfaßt die Erfindung folgende Gegenstände:
- einem Dermatomykose Impfstoff, dadurch gekennzeichnet, daß er antigenes Material von wenigstens einem der folgenden Dermatophyten enthält:
   - Trichophyton verrucosum, insbesondere Trichophyton verrucosum Stamm Nr. VKPGF-931/410 und/oder
   - Trichophyton mentagrophytes, insbesondere Trichophyton mentagrophytes Stamm Nr. VKPGF-930/1032 und /oder
   - Microsporum canis, insbesondere Microsporum canis Stamm Nr. VKPGF-928/1393,
   - Microsporum canis var. obesum, insbesondere Microsporum canis var. obesum Stamm Nr. VKPGF-727/1311 und/oder
   - Microsporum canis var. distortum, insbesondere Microsporum canis var. distortum Stamm Nr. VKPGF-728/120 und/oder
   - Microsporum gypseum, insbesondere Microsporum gypseum Stamm Nr. VKPGF-729/59
   sowie einen physiologisch akzeptablen Träger.
- einen Dermatomykose Impfstoff, insbesondere als Mittel zur Behandlung von Hunden, Katzen und Pferden, dadurch gekennzeichnet, daß der antigenes Material der Dermatophytenstämme Trichophyton verrucosum Nr. VKPGF-931/410, Trichophyton mentagrophytes Nr. VKPGF-930/1032, Trichophyton equinum Stamm Nr. VKPGF-929/381, Trichophyton sarkisovii Nr. VKPGF-551/68, Microsporum canis Nr. VKPGF-928/1393, Microsporum canis var. obesum Nr. VKPGF-727/1311, Microsporum canis var. distortum Nr. VKPGF-728/120 und Microsporum gypseum Nr. VKPGF-729/59 sowie einen physiologisch akzeptablen Träger umfaßt.
- einen Dermatomykose Impfstoff, insbesondere als Mittel zur Behandlung von Rindern, dadurch gekennzeichnet, daß er antigenes Material der Dermatophytenstämme Trichophyton verrucosum Stamm Nr. VKPGF-931/410, Trichophyton mentagrophytes Nr. VKPGF-930/1032 und Trichophyton sarkisovii Nr. VKPGF-551/68 sowie einen physiologisch akzeptablen Träger umfaßt,
- einen wie oben beschriebenen Dermatomykose Impfstoff, dadurch gekennzeichnet, daß er 40 bis 120 Millionen, bevorzugt 90 Millionen Mikrokonidien enthält,
- einen wie oben beschriebenen Dermatomykose Impfstoff, dadurch gekennzeichnet, daß er Thiomersal oder Formaldehyd oder 2-Propyolacton als Inaktivator enthält,
- einen wie oben beschriebenen Dermatomykoseimpfstoff, dadurch gekennzeichnet, daß als physiologisch akzeptablen Träger eine wäßrige Lösung mit 0,2 bis 2,0 Gewichtsprozent fermentiertes, hydrolysiertes Muskelprotein, 5 bis 12 Gewichtsprozent Glucose und 0,1 bis 1,2 Gewichtsprozent Hefeextrakt verwendet wird,
- die Dermatophytenstämme:
   Trichophyton verrucosum Stamm Nr. VKPGF-931/410,
   Trichophyton mentagrophytes Stamm Nr. VKPGF-930/1032,
   Trichophyton equinum Stamm Nr. VKPGF-929/381,
   Microsporum canis Stamm Nr. VKPGF-928/1393,
   Microsporum canis var. obesum Stamm Nr. VKPGF-727/1311,
   Microsporum canis var. distortum Stamm Nr. VKPGF-728/120 und
   Microsporum gypseum Stamm Nr. VKPGF-729/59.
- ein Verfahren zur Herstellung eines Impfstoffes, dadurch gekennzeichnet, daß
   a. antigenes Material aus mindestens einem der folgenden Stämme hergestellt wird
      - Trichophyton verrucosum Nr. VKPGF-931/410,
      - Trichophyton mentagrophytes Nr. VKPGF-551/68,
      - Trichophyton sarkisovii Nr. VKPGF-551/68,
      - Microsporum canis Nr. VKPGF-928/1393,
      - Microsporum canis var. obesum Nr. VKPGF-727/1311,
      - Microsporum canis var. distortum Nr. VKPGF-728/120,
      - Microsporum gypseum Nr. VKPGF-729/59
      und
   b. das antigene Material mit einem physiologisch akzeptablen Träger versetzt wird.
- ein Verfahren wie oben beschrieben, dadurch gekennzeichnet, daß ein Agens, insbesondere Thiomersal, Formaldehyd oder 2-Propyolacton zur Inaktivierung der Dermatophyten zugesetzt wird.

Anhand der folgenden Beispiele wird die Erfindung erläutert.

### Beispiele

### Beispiel 1:

Zur Produktion von 1 l Impfstoff wurden Kulturen der Stämme VKPGF-931/410, 930/1032, 929/381, 551/68, 928/1393, 727/1311, 728/120 und 729/59 auf Agar/Bierwürze bei 26°C für 15 Tage angezogen. Jede Kultur wird in 8 Flaschen ("mattress flasks") angezogen. Die Pilzmasse wird dann abgenommen, homogenisiert und in 200 ml Lösung zu jedem Mischer gegeben. Die benutzte Lösung ist eine wäßrige Lösung mit 1 % fermentiertes, hydrolysiertes Muskelprotein, 10 % Glukose und 1 % Hefeextrakt. Die Konzentration an Mikrokonidien wird auf 90 Millionen pro ml Homogenat gebracht. Nach zwei Tagen werden 125 ml von jeder Suspensionskultur abgenommen und in einem Einzelbehälter vermischt. Impfstoffe können dann durch Vermischung verschiedener Kombinationen der angegebenen Stämme präpariert werden.

Zur Inaktivierung der Homogenatmischung wird Thiomersal direkt im Verhältnis 1:20000 zugegeben. 50 mg Thiomersal wird zu jedem Liter Homogenat zugegeben. Die Zellmischung wird bei Raumtemperatur für zwei Tage aufbewahrt.

Der resultierende Impfstoff wird abgepackt, auf Sterilität, Sicherheit und immunogene Eigenschaften in Übereinstimmung mit akzeptierten Methoden überprüft und bei 4°C gelagert.

Vakzine, die auf diese Art und Weise hergestellt wurden, wurden zur Immunisierung von Tieren benutzt.

Für prophylaktische und therapeutische Zwecke wurde der Impfstoff in folgenden Dosen eingesetzt (Tabelle 8):

**TABELLE 8**

| | | | Dosis (ml) | | |
|---|---|---|---|---|---|
| Tierfamilie | Alter | Injektionsart | prophylaktisch | therpeutisch | |
| Felidae mittelgroße und große Katzen | 1 - 6 Monate | Gluteale Muskeln | 2 bis 5 | 3 bis 6 | |
| | 6 Monate + | Gluteale Muskeln | 3 bis 7 | 4 bis 10 | |
| kleine Katzen | 1 - 5 Monate | Gluteale Muskeln | 1 bis 1,5 | 1 bis 1,5 | |
| | 5 Monate + | Gluteale Muskeln | 1 bis 2 | 1 bis 2 | |
| | Ursidae | 1 - 12 Monate | Gluteale Muskeln | 1 bis 3 | 3 bis 5 |
| | | 12 Monate + | Gluteale Muskeln | 3 bis 5 | 5 bis 6 |
| Procyonidae | 1 - 10 Monate | Gluteale Muskeln | 0,3 bis 0,5 | 0,5 | |
| | 10 Monate + | Gluteale Muskeln | 0,3 bis 0,5 | 0,5 bis 1,0 | |
| Viverridae | 1 - 12 Monate | Gluteale Muskeln | 0,3 bis 0,5 | 0,5 | |
| | 12 Monate + | Gluteale Muskeln | 0,5 bis 1,0 | 0,5 bis 1,0 | |
| Hyaenidae | 1 - 12 Monate | Gluteale Muskeln | 1 bis 3 | 1 bis 3 | |
| | 12 Monate + | Gluteale Muskeln | 3 bis 5 | 5 bis 6 | |
| Canidae | 1 - 10 Monate | Glutaeale und Schultermuskeln | 0,3 bis 0,5 | 0,5 bis 1,0 | |
| | 10 Monate + | | 0,3 bis 1,0 | 0,5 bis 1,0 | |
| Equidae | 3 - 12 Monate | Nackenbereich | 0,3 bis 0,5 | 0,5 bis 1,0 | |
| | 12 Monate + | Nackenbereich | 0,5 | 0,5 bis 1,0 | |
| Tyropodae | 1 - 6 Monate | Schulter- und Nackenbereich | 3 bis 5 | 5 bis 10 | |
| | 6 Monate + | | 5 bis 8 | 7 bis 10 | |
| Bovidae | 1 - 12 Monate | Nackenbereich | 3 bis 5 | 5 bis 10 | |
| | 12 Monate | Nackenbereich | 5 bis 8 | 7 bis 10 | |

### Beispiel 2

Der Impfstoff, hergestellt nach den in Beispiel 1 beschriebenen Methoden, wurde an Labortieren und verschiedenen anderen Tieren auf Effektivität der Prävention und Therapie der Krankheit getestet. Die Ergebnisse sind in Tabelle 9 dargestellt.

### Beispiel 3

Der Impfstoff, der nach Beispiel 1 hergestellt wurde, wurde auch zur Behandlung von Tieren verwendet, die an Dermatomykosen erkrankt waren. Die Ergebnisse in Tabelle 10 dargestellt.

**TABELLE 9**

| Tier | Anzahl | Dosis (cm³) | Effekt |
|---|---|---|---|
| Kaninchen | 10 | 1,0 | Keine Symptome der Krankheit nach Injektion mit virulenten Kulturen der Pilze, T. mentagrophytes, T. verrucosum, T. equinum, M. canis, M. gypseum. |
| Hunde | 5 | 0,3 | |
| Hauskatzen | 3 | 1,0 | |
| Pferde | 5 | 0,5 | Keine Dermatomykosen in Verbindung mit den Pilzen M. canis und T. mentagrophytes nach direktem Kontakt mit erkrankten Tieren. |
| Ponys | 3 | 0,3 | |
| Kamele | 2 | 5,0 | |
| Bären | 2 | 3,0 | |
| Leoparden | 2 | 4,0 | |
| Hyänen | 2 | 2,0 | Keine Dermatomykosen in Verbindung mit den Pilzen M. canis und T. mentagrophytes nach direkten Kontakt mit Infektionsquellen. |
| Serval | 2 | 3,0 | |
| Ozelot | 2 | 2,0 | |
| Löwen | 2 | 3,0 | |
| Tiger | 3 | 7,0 | |
| Nasua | 3 | 0,5 | |
| Zibetkatzen | 2 | 1,0 | |
| Kaninchen | 7 | 1,5 | Keine Symptome der Krankheit nach Infektion mit virulenten Kulturen der Pilze T. sarkisovii und M. gypseum. |
| Hunde | 3 | 0,5 | |
| Hauskatzen | 3 | 1,5 | |
| Schwarze Panther | 2 | 5,0 | Keine Dermatomykosen in Verbindung mit den Pilzen M. canis, T. mentagrophytes und T. verrucosum nach direktem Kontakt mit Infektionsquellen. |
| Tiger | 5 | 7,0 | |
| Gänse | 6 | 3,0 | |
| Bären | 3 | 1,0 | |
| Hunde | 8 | 0,5 | |
| Lamas | 2 | 3,0 | |

**TABELLE 10**

| Tier | Anzahl | Dosis (ml) | Effekt |
|---|---|---|---|
| Schwarze Panther | 5 | 7,0 | Mit Mikrosporie befallen in Verbindung mit dem Pilz M. canis. Heilung fand innerhalb von 12 bis 25 Tagen nach Immunisation statt. |
| Schwarze Panther | 3 | 4,0 | |
| Pferde | 3 | 1,0 | |
| Ponys | 2 | 0,5 | |
| Löwen | 3 | 10 | |
| Tiger | 3 | 10 | |
| Hunde | 4 | 0,5 | |
| Bären | 1 | 5,0 | |
| Hyänen | 1 | 5,0 | |
| Hauskatzen | 15 | 1,5 | Befallen mit Mikrosporie in Verbindung mit dem Pilz M. canis. Heilung fand innerhalb von 10 bis 20 Tagen nach Immunisation statt. |
| Hunde | 5 | 0,5 | |
| Pferde | 5 | 0,7 | |
| Schwarze Panther | 1 | 6,0 | Befallen mit Trichophytie in Verbindung mit dem Pilz T. mentagrophytes. Heilung fand innerhalb von 12 bis 15 Tagen statt. |
| Rotfüchse | 4 | 1,0 | |
| Bären | 2 | 5,0 | |
| Bergschafe | 1 | 7,0 | |
| Pferde | 15 | 1,0 | Befallen mit Mikrosporie in Verbindung mit dem Pilz M. equinum. Heilung fand innerhalb von 12 - 20 Tagen nach Immunisation statt. |

### Literaturverzeichnis

(1) Aisenberg, A.A., Noskow, A.I., Kolovatsky, P.P. "Primenenie Yuglona v Veterinarii" in Scientific and Technical Information Bulletin of the State Scientific Control Committee under the Moldavian Council of Ministers (1958), p. 88.
(2) UdSSR Patent Nr. 548947 (1976).

## Patentansprüche

1. Impfstoff, dadurch gekennzeichnet, daß er antigenes Material von Wenigstens einem der folgenden inaktivierten (abgetöteten) Dermatophyten-Stämme enthält:
Trichophyton verrucosum Nr. VKPGF 931/410 (DSM-Nr. 7277) oder
Trichophyton mentagrophytes Nr. VKPGF 930/1032 (DSM-Nr. 7279) oder
Trichophyton equinum Nr. VKPGF 929/381 (DSM-Nr. 7276) oder
Microsporum canis Nr. VKPGF 928/1393 (DSM-Nr. 7281) oder
Microsporum canis var. obesum Nr. VKPGF 727/1311 (DSM-Nr. 7280) oder
Microsporum canis var. distortum Nr. VKPGF 728/120 (DSM-Nr. 7275) oder
Microsporum gypseum Nr. VKPGF 729/59 (DSM-Nr. 7274) enthält.

2. Impfstoff gemäß Anspruch 1, dadurch gekennzeichet, daß er antigenes Material einer Kombination der in Anspruch 1 genannten Dermatophytenstämme enthält.

3. Impfstoff gemäß Anspruch 1 oder 2, dadurch gekennzeichnet, daß er zusätzlich antigenes Material von Trichophyton sarkisovii Nr. VKPGF 551/68 (DSM-Nr.7278) enthält.

4. Impfstoff gemäß einem der Ansprüche 1 bis 3, dadurch gekennzeichnet, daß er antigenes Material der folgenden Dermatophyten-Stämme enthält: Trichophyton verrucosum Nr. VKPGF 931/410 (DSM-Nr. 7277) und Trichophyton mentagrophytes Nr. VKPGF 930/1032 (DSM-Nr. 7279) und Trichophyton sarkisovii Nr. VKPGF 551/68 (DSM-Nr.7278).

5. Impfstoff gemäß einem der Ansprüche 1 bis 3, dadurch gekennzeichnet, daß er antigenes Material der folgenden Dermatophyten-Stämme enthält:
Trichophyton verrucosum Nr. VKPGF 931/410 (DSM-Nr. 7277) und
Trichophyton mentagrophytes Nr. VKPGF 930/1032 (DSM-Nr. 7279) und
Trichophyton equinum Nr. VKPGF 929/381 (DSM-Nr. 7276) und
Microsporum canis Nr. VKPGF 928/1393 (DSM-Nr. 7281) und
Microsporum canis var. obesum Nr. VKPGF 727/1311 (DSM-Nr. 7280) und
Microsporum canis var. distortum Nr. VKPGF 728/120 (DSM-Nr. 7275) und
Microsporum gypseum Nr. VKPGF 729/59 (DSM-Nr. 7274) enthält.

6. Impfstoff gemäß einem der Ansprüche 1 bis 5, dadurch gekennzeichnet, daß es sich bei dem antigenen Material um inaktivierte reine Dermatophytenkulturen handelt.

7. Impfstoff gemäß einem der Ansprüche 1 bis 6, dadurch gekennzeichnet, daß die Dermatophytenkultur mit Thiomersal, 2-Propyolacton oder Formaldehyd inaktiviert wurde.

8. Impfstoff gemäß einem der Ansprüche 1 bis 7, dadurch gekennzeichnet, daß die Dermatophytenkultur homogenisiert wurde.

9. Impfstoff gemäß einem der Ansprüche 1 bis 8, dadurch gekennzeichnet, daß er 40 bis 120 Millionen Mikrokonidien pro ml enthält.

10. Impfstoff gemäß einem der Ansprüche 1 bis 9, dadurch gekennzeichnet, daß er ein Adjuvant enthält.

11. Impfstoff gemäß einem der Ansprüche 1 bis 10, dadurch gekennzeichnet, daß er einen physiologisch akzeptablen Träger enthält.

12. Impfstoff gemäß einem der Ansprüche 1 bis 11, dadurch gekennzeichnet, daß er als physiologisch akzeptablen Träger eine wäßrige Lösung mit 0,2 bis 2 Gewichtsprozent fermentiertes, hydrolysiertes Muskelprotein, 5 bis 12 Gewichtsprozent Glukose und 0,1 bis 1,2 Gewichtsprozent Hefeextrakt enthält.

13. Trichophyton verrucosum Nr. VKPGF 931/410 (DSM-Nr. 7277).

14. Trichophyton mentagrophytes Nr VKPGF 930/1032 (DSM-Nr. 7279).

15. Trichophyton equinum Nr. VKPGF 929/381 (DSM-Nr. 7276).

16. Microsporum canis Nr. VKPGF 928/1393 (DSM-Nr. 7281).

17. Microsporum canis var. obesum Nr. VKPGF 727/1311 (DSM-Nr. 7280).

18. Microsporum canis var. distortum Nr. VKPGF 728/120 (DSM-Nr. 7275).

19. Microsporum gypseum Nr. VKPGF 729/59 (DSM-Nr. 7274).

20. Verwendung eines Impfstoffes gemäß einem der Ansprüche 1 bis 12, zur Herstellung eines Arzneimittels zur Prophylaxe und/oder Therapie von Dermatomykosen.

21. Verwendung des Impfstoffes gemäß einem der Ansprüche 1 bis 12, zur Herstellung eines Arzneimittels zur Bildung von Immunität und/oder zur Resistenz gegen Infektionen von Dermatophyten.

22. Verfahren zur Herstellung eines Impfstoffes gemäß einem der Ansprüche 1 bis 12, dadurch gekennzeichnet, daß die Dermatophytenkultur in einer wäßrigen Lösung homogenisiert, etwa zwei Tage inkubiert und dann inaktiviert wird.

23. Verfahren gemäß Anspruch 22, dadurch gekennzeichnet, daß die Mischung nach der Homogenisation auf 40 bis 120 Millionen Mikrokonidien pro ml eingestellt wird.

24. Verfahren gemäß Anspruch 22 oder 23, dadurch gekennzeichnet, daß die Mischung nach der Homogenisation auf etwa 90 Millionen Mikrokonidien pro ml eingestellt wird..

25. Verfahren gemäß einem der Ansprüche 22 bis 24, dadurch gekennzeichnet, daß die Inaktivierung mittels Thiomersal, 2-Propyolacton oder Formaldehyd durchgeführt wird.

26. Verfahren gemäß einem der Ansprüche 22 bis 25, dadurch gekennzeichnet, daß die Kultur vor dem Homogenisationsschritt auf einem festen Nährboden kultiviert wird.

## Claims

1. Vaccine, characterised in that it contains antigenic material from at least one of the following inactivated (killed) dermatophyte strains:
Trichophyton verrucosum No. VKPGF 931/410 (DSM No. 7277) or
Trichophyton mentagrophytes No. VKPGF 930/1032 (DSM No. 7279) or
Trichophyton equinum No. VKPGF 929/381 (DSM No. 7276) or
Microsporum canis No. VKPGF 928/1393 (DSM No. 7281) or
Microsporum canis var. obesum No. VKPGF 727/1311 (DSM No. 7280) or
Microsporum canis var. distortum No. VKPGF 728/120 (DSM No. 7275) or
Microsporum gypseum No. VKPGF 729/59 (DSM No. 7274).

2. Vaccine according to claim 1, characterised in that it contains antigenic material from a combination of the dermatophyte strains recited in claim 1.

3. Vaccine according to claim 1 or 2, characterised in that it additionally contains antigenic material from Trichophyton sarkisovii No. VKPGF 551/68 (DSM No. 7278).

4. Vaccine according to one of claims 1 to 3,
characterised in that it contains antigenic material from the following dermatophyte strains:
Trichophyton verrucosum No. VKPGF 931/410 (DSM No. 7277) and
Trichophyton mentagrophytes No. VKPGF 930/1032 (DSM No. 7279) and
Trichophyton sarkisovii No. VKPGF 551/68 (DSM No. 7278).

5. Vaccine according to one of claims 1 to 3,
characterised in that it contains antigenic material from the following dermatophyte strain:
Trichophyton verrucosum No. VKPGF 931/410 (DSM No. 7277) and
Trichophyton mentagrophytes No. VKPGF 930/1032 (DSM No. 7279) and
Trichophyton equinum No. VKPGF 929/381 (DSM No. 7276) and
Microsporum canis No. VKPGF 928/1393 (DSM No. 7281) and
Microsporum canis var. obesum No. VKPGF 727/1311 (DSM No. 7280) and
Microsporum cans var. distortum No. VKPGF 728/120 (DSM No. 7275) and
Microsporum gypseum No. VKPGF 729/59 (DSM No. 7274).

6. Vaccine according to one of claims 1 to 5,
characterised in that the antigenic material consists of inactivated pure dermatophyte cultures.

7. Vaccine according to one of claims 1 to 6,
characterised in that the dermatophyte culture has been inactivated rich thiomersal, 2-propyolactone or formaldehyde.

8. Vaccine according to one of claims 1 to 7,
characterised in that the dermatophyte culture has been homogenised.

9. Vaccine according to one of claims 1 to 8,
characterised in that it contains 40 to 120 million microconidia per ml.

10. Vaccine according to one of claims 1 to 9,
characterised in that it contains an adjuvant.

11. Vaccine according to one of claims 1 to 10,
characterised in that it contains a physiologically acceptable carrier.

12. Vaccine according to one of claims 1 to 11,
characterised in that it contains, as physiologically acceptable carrier, an aqueous solution containing 0.2 to 2% by weight of fermented hydrolysed muscle protein, 5 to 12% by weight of glucose and 0.1 to 1.2% by weight of yeast extract.

13. Trichophyton verrucosum No. VKPGF 931/410 (DSM No. 7277).

14. Trichophyton mentagrophytes No. VKPGF 930/1032 (DSM No. 7279).

15. Trichophyton equinum No VKPGF 929/381 (DSM No. 7276).

16. Microsporum canis No. VKPGF 928/1393 (DSM No. 7281).

17. Microsporum canis var. obesum No. VKPGF 727/1311 (DSM No. 7280).

18. Microsporum canis var. distortum No. VKPGF 728/120 (DSM No. 7275).

19. Microsporum gypseum No. VKPGF 729/59 (DSM No. 7274).

20. Use of a vaccine according to one of claims 1 to 12 for preparing a pharmaceutical composition for the prevention and/or treatment of dermatomycoses.

21. Use of the vaccine according to one of claims 1 to 12 for preparing a pharmaceutical composition for building up immunity and/or resistance against infections by dermatophytes.

22. Process for preparing a vaccine according to one of claims 1 to 12, characterised in that the dermatophyte culture is homogenized in an agueous solution, incubated for about two days and then inactivated.

23. Process according to claim 22, characterised in that, after homogenisation, the mixture is adjusted to 40 to 120 million microconidia per ml.

24. Process according to claim 22 or 23, characterised in that after homogenisation the mixture is adjusted to about 90 million microconidia per ml.

25. Process according to one of claims 22 to 24, characterised in that the inactivation is carried out using thiomersal, 2-propyolactone or formaldehyde.

26. Process according to one of claims 22 to 25, characterised in that, before the homogenisation step, the culture is grown on a solid nutrient base.

## Revendications

1. Vaccin caractérisé en ce qu'il contient un matériel antigénique d'au moins l'une des souches de dermatophytes inactivées (tuées) suivantes:
Trichophyton verrucosum n°. VKPGF 931/410 (n°. DSM 7277) ou Trichophyton mentagrophytes n°. VKPGF 930/1032 (n°. DSM 7279) ou
Trichophyton equinum n°. VKPGF 929/381 (n°. DSM 7276) ou
Microsporum canis n°. VKPGF 928/1393 (n°. DSM 7281) ou
Microsporum canis var. obesum n°. VKPGF 727/1311 (n°. DSM 7280) ou
Microsporum canis var. distortum n°. VKPGF 728/120 (n°. DSM 7275) ou
Microsporum gypseum n°. VKPGF 729/59 (n°. DSM 7274).

2. Vaccin selon la revendication 1 caractérisé en ce qu'il contient un matériel antigénique d'une combinaison des souches de dermatophytes citées dans la revendication 1.

3. Vaccin selon la revendication 1 ou 2 caractérisé en ce qu'il contient en outre un matériel antigénique de Trichophyton sarkisovii n°. VKPGF 551/68 (n°. DSM 7278).

4. Vaccin selon l'une des revendications 1 à 3 caractérisé en ce qu'il contient un matériel antigénique des souches de dermatophytes suivantes: Trichophyton verrucosum n°. VKPGF 931/410 (n°. DSM 7277) et Trichophyton mentagrophytes n°. VKPGF 930/1032 (n°. DSM 7279) et
Trichophyton sarkisovii n°. VKPGF 551/68 (n°. DSM 7278).

5. Vaccin selon l'une des revendications 1 à 3 caractérisé en ce qu'il contient un matériel antigénique des souches de dermatophytes suivantes:
Trichophyton verrucosum n°. VKPGF 931/410 (n°. DSM 7277) et
Trichophyton mentagrophytes n°. VKPGF 93011032 (n°. DSM 7279) et
Trichophyton equinum n°. VKPGF 929/381 (n°. DSM 7276) et
Microsporum canis n°. VKPGF 928/1393 (n°. DSM 7281) et
Microsporum canis var. obesum n°. VKPGF 727/1311 (n°. DSM 7280) et
Microsporum canis var. distortum n°. VKPGF 728/120 (n°. DSM 7275) et
Microsporum gypseum n°. VKPGF 729/59 (n°. DSM 7274).

6. Vaccin selon l'une des revendications 1 à 5 caractérisé en ce qu'il s'agit, concernant le matériel antigénique, de cultures de dermatophytes pures inactivées.

7. Vaccin selon l'une des revendications 1 à 6 caractérisé en ce que la culture de dermatophytes a été inactivées avec le thiomersal, la 2-propiolactone ou le formaldéhyde.

8. Vaccin selon l'une des revendications 1 à 7 caractérisé en ce que la culture de dermatophytes a été homogénéisée.

9. Vaccin selon l'une des revendications 1 à 8 caractérisé en ce qu'il contient 40 à 120 millions de microconidies par ml.

10. Vaccin selon l'une des revendications 1 à 9 caractérisé en ce qu'il contient un adjuvant.

11. Vaccin selon l'une des revendications 1 à 10 caractérisé en ce qu'il contient un véhicule physiologiquement acceptable.

12. Vaccin selon l'une des revendications 1 à 11 caractérisé en ce qu'il contient comme véhicule physiologiquement acceptable une solution aqueuse contenant 0,2 à 2 % en masse de protéine musculaire hydrolysée fermentée, 5 à 12 % en masse de qlucose et 0,1 à 1,2 % en masse d'extrait de levure.

13. Trichophyton verrucosum n°. VKPGF 931/410 (n°. DSM 7277).

14. Trichophyton mentagrophytes n°. VKPGF 930/1032 (n°. DSM 7279).

15. Trichophyton equinum n°. VKPGF 929/381 (n°. DSM 7276).

16. Microsporum canis n°. VKPGF 928/1393 (n°. DSM 7281).

17. Microsporum canis var. obesum n°. VKPGF 727/1311 (n°. DSM 7280).

18. Microsporum canis var. distortum n°. VKPGF 728/120 (n°. DSM 7275).

19. Microsporum qypseum n°. VKPGF 729/59 (n°. DSM 7274).

20. Utilisation d'un vaccin selon l'une des revendications 1 à 12 pour la préparation d'un médicament pour la prophylaxie et/ou la thérapie des dermatomycoses.

21. Utilisation d'un vaccin selon l'une des revendications 1 à 12 pour la préparation d'un médicament pour la formation d'une immunité et/ou pour la résistance contre les infections de dermatophytes.

22. Procédé de préparation d'un vaccin selon l'une des revendications 1 à 12 caractérisé en ce que la culture de dermatophytes est homogénéisée dans une solution aqueuse, incubée pendant environ deux jours puis inactivée.

23. Procédé selon la revendication 22 caractérisé en ce que le mélange est ajusté à 40 à 120 millions de microconidies par ml après l'homogénéisation.

24. Procédé selon la revendication 22 ou 23 caractérisé en ce que le mélange est ajusté à environ 90 millions de microconidies par ml après l'homogénéisation.

25. Procédé selon l'une des revendications 22 à 24 caractérisé en ce que l'inactivation est réalisée au moyen de thiomersal, de 2-propiolactone ou de formaldéhyde.

26. Procédé selon l'une des revendications 22 à 25 caractérisé en ce que la culture est cultivée sur un milieu nutritif solide avant l'étape d'homogénéisation.
